Europäisches Patentamt

European Patent Office

Office européen des brevets

(19)

(11) Publication number: **0 269 408**
**A2**

(12) **EUROPEAN PATENT APPLICATION**

(21) Application number: **87310341.0**

(51) Int. Cl.⁴: **A 61 K 37/02**

(22) Date of filing: **24.11.87**

(30) Priority: **26.11.86 US 935445   03.11.87 US 116101**

(43) Date of publication of application:
**01.06.88 Bulletin 88/22**

(84) Designated Contracting States:
**AT BE CH DE ES FR GB GR IT LI LU NL SE**

(71) Applicant: **GENENTECH, INC.**
**460 Point San Bruno Boulevard**
**South San Francisco California 94080  (US)**

(72) Inventor: **Shepard, Harold Michael**
**35 Delano Street**
**San Francisco California 94112  (US)**

(74) Representative: **Armitage, Ian Michael et al**
**MEWBURN ELLIS & CO. 2/3 Cursitor Street**
**London EC4A 1BQ  (GB)**

(54) **TGF-Beta in the treatment of inflammatory disorders.**

(57) Methods and compositions are provided for the treatment of inflammatory disorders such as rheumatoid arthritis, inflammatory bowel disease, systemic lupus erythematosus and the like. Therapeutically effective doses of Transforming Growth Factor-β are administered to patients in order to ameliorate these disorders.

## Description

### TGF-β IN THE TREATMENT OF INFLAMMATORY DISORDERS

This invention relates to the treatment of inflammatory disorders.

Inflammatory responses associated with various immune disorders are typically divided into four classes. Class I responses are reaginic or allergic reactions characterizing atopy or anaphylaxis. Class II responses are dependent upon cytotoxic antibody and are associated, for example, with autoimmune hemolytic anemia and thrombocytopenia attendant to systemic lupus erythematosus (SLE). Class III responses are characterized by chronic generation of immune complexes containing IgG and/or IgM. Class IV responses, associated with delayed hypersensitivity, are mediated by cytokines and T-lymphocytes and are typically found in tuberculosis, sarcoidosis, polymyositis, granulomatosis and vasculitis. This invention is particularly concerned with inappropriate or aberrant class II and class III responses. These classes are generally associated with inflammatory sequelae dependent upon chronic immune dysfunction including constitutive production of soluble mediators including cytokines, immunoglobulins and complement components.

Rheumatoid arthritis (RA) is a serious class III disorder. Synovitis is the characteristic tissue reaction in RA, wherein the normally thin connective tissue is replaced by hyperproliferating synovial fibroblasts, and the synovium is invaded by an infiltrate of lymphocytes, macrophages and other immune cells. The turnover of complement components is increased in rheumatic synovial fluid, and complement cleavage products, especially C5a, are present in rheumatoid synovial fluid. This is significant because C5a and other derivatives of complement contribute to the activation of immune cells and the production of inflammatory cytokines. The fluid also contains hydrolytic enzymes (including collagenase) derived from inflammatory cells, together with kinins and LTB4.

Various mechanisms have been proposed to account for the degree of tissue destruction characteristic of rheumatoid arthritis. These mechanisms involve a complex interplay of activation and suppression mechanisms which modulate the type and magnitude of the inflammatory response in RA. These mechanisms involve arachidonic acid metabolites, especially prostaglandin E2 produced by leukocytes and rheumatoid synovia, biologically active amines such as histamine and serotonin, complement cleavage products, eosinophil chemotactic factors, kinins, interleukins and proteolytic enzymes, especially collagenase or procollagenase activating enzymes.

One commonly accepted postulate for induction of RA holds that an undefined antigen is chronically deposited in the synovium where it is phagocytized by the Type A synovial cells. Presentation of the antigen to B cells induces their differentiation to plasma cells, which then produce antibodies, rheumatoid factors and cytokines. Antigen activation of T lymphocytes triggers lymphokine synthesis, blastogenesis and the subsequent generation of antigen-specific helper and cytotoxic T-cells. The combination of antibody with antigen, as well as combination of antigen-antibody complexes with rheumatoid factors, or self-association of rheumatoid factors, activates complement as well as the kinin-forming system by means of activating Hageman factor. This results in the production of proinflammatory products such as C5a, arachidonic acid metabolites, kinins, and fibrinopeptides, which diffuse into the synovial fluid and to synovial blood vessels. These agents enhance vascular permeability and attract polymorphonuclear leukocytes and macrophages. Polymorphonuclear leukocytes ingest the abundant immune complexes in the fluid, release lysosomal and other destructive enzymes, and generate superoxide anions. This causes destruction of hyaluronate polymers in the joint fluid, as well as injury to cartilage. Cytokine production in the synovium leads to the further accumulation of macrophages, other immune cells and rheumatoid synovial fibroblasts. These cell types can all contribute further to the cycle of immune cell recruitment, activation and production of immune mediators (e.g., cytokines) which is characteristic of RA and leads to the perpetuation of the inflammatory state and the resulting tissue destruction.

The unique structure of the joint space is important, as enzymes present in synovial fluid or released and synthesized locally by the cells constituting the proliferative synovial lesion contribute to the pathology evident in articular structures. The cartilage-degrading lysosomal enzymes collagenase and elastase are primarily derived from inflammatory cells (immune cells and rheumatoid synovial fibroblasts). Proteinases released by dying cells may aid in superficial cartilage destruction by virtue of their role in uncrosslinking collagen fibrils, thus increasing their susceptibility to enzymatic degradation. Macrophages in the synovium produce prostaglandins, hydrolytic enzymes, collagenase, plasminogen activator, and IL-1. Collagenase synthesis by macrophages or rheumatoid synovial fibroblasts is greatly enhanced in the presence of IL-1 and other inflammatory proteins. In addition to collagenases, lysosomal proteinases can degrade aggregates of proteoglycans, and, once released from cartilage, these solubilized components are then sensitive to further enzymatic attack.

The net effect, resulting from either persistence of antigen or disordered regulation of T and B cell activation, is chronic inflammatory response in the synovium. Continued cellular proliferation and influx lead to synovial proliferation and its invasion into surrounding structures. Diffusion of collagenase, PGEs, hydrolytic enzymes, and cytokines into cartilage and bone results in erosion of these tissues. Rheumatoid arthritis thus illustrates the devastating local tissue destruction that results from chronic inflammatory reactions produced by delayed hypersensitivity types of immune responses.

Current therapy for RA includes bed rest, application of heat, and drugs. Salicylate is the currently preferred drug, particularly as other alternatives such as immunosuppressive agents and adrenocorticosteroids can cause greater morbidity than the underlying disease itself. Nonsteroidal anti-inflammatory drugs are available, and many of them have effective analgesic, antipyretic and anti-inflammatory activity in RA patients. These include indomethacin, phenylbutazone, phenylacetic acid derivatives such as ibuprofen and fenoprofen, naphthalene acetic acids (naproxen), pyrrolealkanoic acid (tometin), indoleacetic acids (sulindac), halogenated anthranilic acid (meclofenamate sodium), piroxicam, zomepirac and diflunisal. These are not generally considered any more effective than aspirin, but may be better tolerated by some patient classes.

Other drugs for use in RA include antimalarials such as chloroquine, gold salts and penicillamine. These alternatives frequently produce severe side effects, including retinal lesions and kidney and bone marrow toxicity. Immunosuppressive agents such as methotrexate have been used only in the treatment of severe and unremitting RA because of their toxicity. Corticosteroids also are responsible for undesirable side effects and are not well tolerated in many RA patients.

Methods and compositions are needed for the effective therapy of chronic inflammatory diseases, in particular for the therapy of responses associated with ulcerative colitis (inflammatory bowel disease), RA or SLE, which are free of adverse side effects.

The present invention relates to the administration of a therapeutically effective dose of transforming growth factor-β to a patient exhibiting an inflammatory disorder.

The invention provides for the use of TGF-β in preparing medicaments for such treatments, and to novel formulations and pharmaceutical preparations for such treatment.

In the drawings:

Figure 1 shows the inhibitory effect of TGF-β on human peripheral blood mononuclear cells in vitro.

Figure 2 illustates the inhibition of allospecific cytotoxic cell generation in the mixed lymphocyte reaction.

Figure 3 shows the N-terminii for selected forms of TGF-β. The letters h, p and b stand for human, porcine and bovine, respectively. Non-homologous residues are designated by a period over the residue in question.

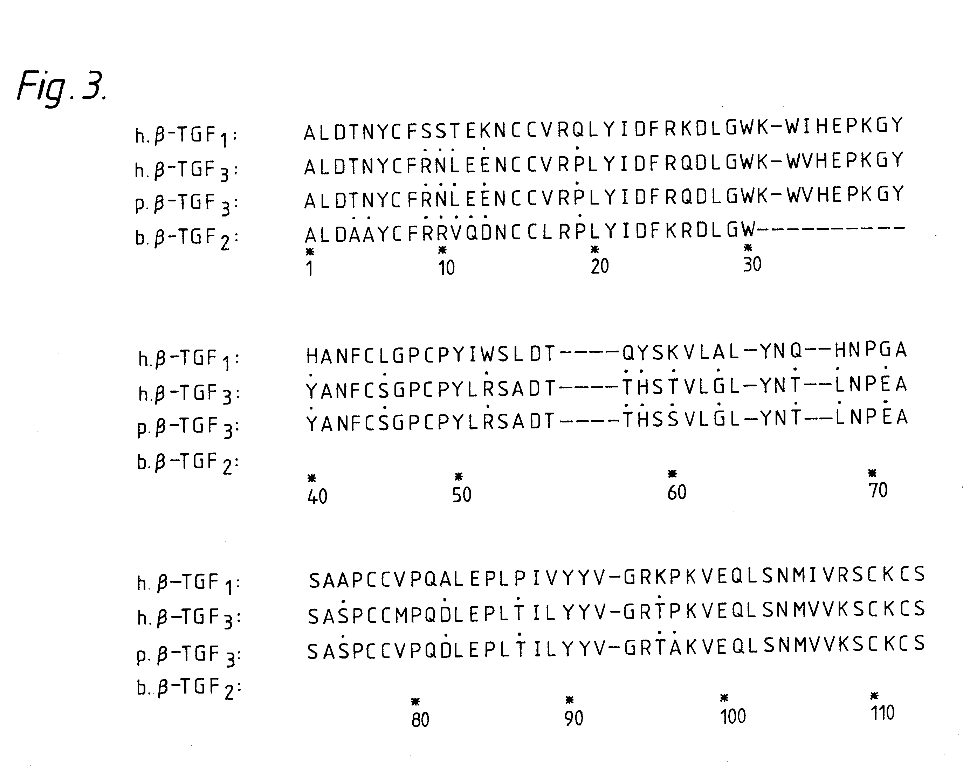

Transforming growth factor-β (TGF-β) is a two-chain molecule containing two identical 112 residue polypeptide chains linked by disulfide bonds. The molecular mass of this dimer is about 25 KDal. Biologically active TGF-β is defined as a molecule capable of inducing anchorage independent growth of target cell lines or rat fibroblasts in in vitro cell culture, when added together with EGF or TGF-α as a co-factor. Suitable methods are known for purifying TGF-β from platelets or placenta, for producing it in recombinant cell culture and for determining its activity. See for example R. Derynck et al., "Nature" 316:701 (1985) and copending U.S.S.N.s 715,142; 500,832; 500,833; 07/25,423 and 500,927, the entire contents thereof being expressly incorporated by reference. Since TGF-β is not species specific it is within the scope hereof to employ TGF-β from animals other than humans, for example porcine or bovine sources. At the present time three highly homologous forms of TGF-β have been identified, TGF-$\beta_1$, TGF-$\beta_2$ and TGF-$\beta_3$. N-terminii for these forms are set forth in Fig. 3. Reference to TGF-β herein will be understood as reference to any one of the three identified forms as well as others identified in the future, their alleles and predeterminied amino acid sequence variants, so long as they are effective in the method described herein.

TGF-β has been shown to have numerous regulatory actions on a wide variety of both normal and neoplastic cells. Recent studies indicate an important role for TGF-β in cells of the immune system (J. Kehrl et al., "J. Exp. Med." 163:1037 [1986]; H-J. Ristow, "Proc. Natl. Acad. Sci. U.S.A." 83:5531 [1986]; and A. Rook et al. , "J. Immunol." 136:3916[1986]) and connective tissue (M. Sporn et al. "Science" 219:1329 [1983]; R. Ignotz et al. "J. Biol. Chem." 261:4337 [1986]; J. Varga et al., "B.B.Res.Comm." 138:974 [1986]; A. Roberts et al., Proc. Natl. Acad. Sci. USA" 78:5339 [1981]; A. Roberts et al., "Fed. Proc." 42:2621 [1983]; and A. Roberts et al., "Proc. Natl. Acad. Sci. U.S.A." 83:4167 [1986]), as well as epithelia (T. Masui et al., "Proc. Natl. Acad. Sci. U.S.A" 83:2438 [1986] and G. Shipley et al. "Cell" 46:2068 [1986]). TGF-β is multifunctional, since it can either stimulate or inhibit cell proliferation, can either stimulate or inhibit differentiation, and can either stimulate or inhibit other critical processes in cell function (M. Sporn, "Science" 233:532 [1986]). U.S.S.N. 500,833 relates to the use of TGF-β to repair tissue in animals, in particular for use in accelerating wound healing by stimulating cell proliferation.

The multifunctional activity of TGF-β is modulated by the influence of other growth factors present together with the TGF-β. Roberts et al. report that TGF-β can function as either an inhibitor or an enhancer of anchorage-independent growth, depending on the particular set of growth factors, e.g., EGF or TGF-α, operant in the cell together with TGF-β ("Proc. Natl. Acad. Sci. U.S.A." 82:119 [1985]). According to Brinkerhoff et al., "Arthritis and Rheumatism" 26:1370 (1983), TGF-β can act in concert with EGF to cause proliferation and piling up of normal (but not rheumatoid) synovial cells, thus raising "the possibility that these factors may play a [destructive] role in rheumatoid arthritis and other proliferative but nonmalignant diseases as well". Furthermore, Chua et al. ("J. Biol. Chem." 260:5213-5216 [1983]) reported that TGF-β induced collagenase secretion in human fibroblast cultures, and A. Tashjian et al. observed that TGF-β stimulated the release of prostaglandins and mobilization of calcium ("Proc. Natl. Acad. Sci. USA" 82: 4535 [1985]). Brinkerhoff, Tashjian and Chua would have taught away from the use of TGF-β to treat inflammatory conditions, the former by observing that TGF-β may be responsible for the hyperproliferation of synovial cells that is characteristic of RA, and Chua and Tashjian et al. for suggesting that TGF-β would induce substances thought to be directly responsible for the inflammation and damage to cartilage and other tissues which is associated

**0 269 408**

with RA. TGF-β also has been reported to inhibit endothelial regeneration (R. Heimark et al., "Science" 233:1078 [1986]). Thus, the inventor's decision to investigate the value of TGF-β in the treatment of inflammatory disease flew in the face of the prior literature.

The diseases or disorders to be treated with TGF-β in accordance with this invention include those which fall within Classes II and III as described above, in particular inflammatory bowel disease, SLE and RA. These disorders are characterized by severe inflammation accompanied by unusually elevated collagenase production and tissue destruction, and are generally thought to have their origin in an aberrant immune response of an unresolved and poorly understood nature.

The TGF-β compositions to be used in the therapy will be formulated and dosed in a fashion consistent with good medical practice taking into account the disorder to be treated, the condition of the individual patient, the site of delivery of the TGF-β, the method of administration and other factors known to practitioners.

TGF-β is prepared for administration by mixing TGF-β at the desired degree of purity with physiologically acceptable carriers, i.e., carriers which are nontoxic to recipients at the dosages and concentrations employed. Ordinarily, this will entail combining TGF-β with buffers, low molecular weight (less than about 10 residues) polypeptides, proteins, amino acids, carbohydrates including glucose or dextrans, chelating agents such as EDTA, and other excipients. TGF-β for use in therapeutic administration must be sterile. This is readily accomplished by sterile filtration through (0.2 micron) membranes. TGF-β ordinarily will be stored as an aqueous solution since it is highly stable to thermal and oxidative denaturation, although lyophilized formulations for reconstitution are acceptable.

Generally, where the disorder permits, one should formulate and dose the TGF-β for site-specific delivery. This is convenient in the case of RA and inflammatory bowel disease. In the former case, TGF-β is formulated into a sterile sustained release composition suitable for injection into the synovial fluid or implantation into the synovial lining or capsule. In the case of inflammatory bowel disease, the TGF-β is formulated into suppositories with pharmaceutically acceptable oleaginous substances as is generally known in the art.

Sustained release formulations will be selected from the classes of microcapsular particles and implantable articles. Liposomal encapsulation is not preferred for injection directly into the synovial cavity in RA because it entails the introduction of lipid into the joint. However, liposomal encapsulation is suitable for implantation of sustained release TGF-β into the synovial capsule. For the treatment of RA using sustained-release TGF-β, the TGF-β is preferably incorporated into a biodegradable matrix or microcapsule. A suitable material for this purpose is a polylactide, although other polymers of poly (α-hydroxycarboxylic acids), such as poly-D-(-)-3-hydroxybutyric acid (EP 133,988A), can be used. Other biodegradable polymers include poly(lactones), poly(acetals), poly(orthoesters) or poly(orthocarbonates). The initial consideration here must be that the carrier itself, or its degradation products, is nontoxic in the target tissue and will not further aggravate the disease. This can be determined by routine screening in animal models of the target disorder or, if such models are unavailable, in normal animals. For examples of sustained release compositions, see U.S. patent 3,773,919, EP 58,481A, U.S. patent 3,887,699, EP 158,277A, Canadian patent 1176565, U. Sidman et al., "Biopolymers" 22:547 [1983], and R. Langer et al., "Chem. Tech." 12:98 [1982].

The dosage to be employed is dependent upon the factors described above. As a general proposition, the TGF-β should be formulated and delivered to the target site or tissue at a dosage capable of establishing in the tissue a TGF-β level greater than about 0.1 ng/cc. Typically, the TGF-β concentrations should range about from 0.5 to 15 ng/cc. These intra-tissue concentrations should be maintained if possible by continuous infusion, sustained release or injection at empirically determined frequencies. The preferred concentration in synovial fluid is about from 0.1 to 10 ng/ml of synovial fluid. Since RA synovial fluid can be present in amounts up to 50 ml, for example in a knee joint, the actual amount of TGF-β will depend upon its dilution into the synovial fluid as well as the empirically determined losses of TGF-β to endogenous proteases and leakage into the general circulation. Thus it is best to evaluate the efficacy of the initial dosage protocol by withdrawing synovial fluid samples for TGF-β assay during early stages of treatment in order to determine the proper dosage regimen.

Preferably TGF-β is not administered with other growth factors such as EGF or TGF-α. However, it is within the scope hereof to combine the TGF-β therapy with other novel or conventional therapies for the disorders in question. For example, in the case of RA the TGF-β therapy may be delivered in concert with other anti-inflammatory substances such as salicylate, nonsteroidal anti-inflammatory drugs, penicillamine, gold salts, TNF-α or TNF-β antagonists (described in compending U.S.S.N. 898,272), γ-interferon antagonists and/or IL-1 antagonists. It is not necessary that such cotreatment drugs be included in the TGF-β compositions per se, although this will be convenient where such drugs are proteinaceous such as in the case of antagonists to the activity of γ-interferon, TNF-α, Il-1 and TNF-β (amino acid sequence variants or neutralizing antibodies).

The invention will be more fully understood by reference to the following examples. They should not, however, be construed as limiting the scope of the invention. All literature citations are incorporated by reference.

EXAMPLE 1

TGF-β Suppression of Peripheral Blood Mononuclear Cell Immune Responses

The purpose of this example was to examine the influence of TGF-β on specific immune response of human peripheral blood mononuclear cells (PMBC) in vitro. The results demonstrate that TGF-β can inhibit tetanus

4

toxoid-induced PBMC proliferative activities in a dose related manner. Further, the addition of TGF-β to mixed lymphocyte cultures results in the suppression of specific cytotoxic cell function tested against targets of the stimulator cell type. The materials and methods used in this example were as follows:

TGF-β: Porcine platelet-derived TGF-β at 96 percent purity was purchased (R+D Systems, Inc., Minneapolis, MN) in a lyophilized form and suspended in 4 mM HCl solution prior to use.

Cell separation: Using heparinized syringes, blood was collected from healthy donors, mixed with an equal volume of saline and layered onto Ficoll-Hypaque gradients (sp. gr. 1.08). After centrifugation at 400 x g for 40 min. at room temperature, peripheral blood mononuclear cells (PBMC) isolated at the plasma-Ficoll interface were removed, washed three times in Hanks balanced salt solution and resuspended in complete culture medium. Cell viability as determined by trypan blue exclusion was consistently $\geq 95$ percent.

Culture medium: RPMI 1640 medium purchased from Grand Island Biological Co. (GIBCO, Grand Island, NY) was supplemented with 2 mM L-glutamine, 500 units/ml penicillin, 500 μg/ml streptomycin (GIBCO) and 10 percent autologous plasma for use in the tetanus toxoid stimulation assay. For mixed lymphocyte cultures, RPMI 1640 medium was supplemented with 10 percent heat inactivated fetal bovine serum (Hyclone, Logan, UT), 2 mM L-glutamine, 0.1 mM non-essential amino acids, 10 mM HEPES, antibiotics (GIBCO) and $5 \times 10^{-5}$ M 2-mercaptoethanol (Sigma, St. Louis, MO).

Tetanus toxoid stimulation assay: Preliminary dose-response studies demonstrated that tetanus toxoid (TT) preparation (obtained from Dr. A.V. Muchmore, National Cancer Institute, Bethesda, MD) diluted 1:300 induced optimal PBMC proliferative response after six days of incubation. PBMC cultures were established in quadruplicate in flat-bottom microliter plates (Beckton Dickinson, Pittsburgh, PA) with $10^5$ cells in 200 μl medium in each well in the presence (stimulated) or absence (unstimulated) of TT. The effects of TGF-β were measured in cultures prepared in parallel. All cultures were incubated at 37°C in air with 5 percent $CO_2$ for six days, the time at which peak responses occurred. Six hours prior to harvesting, the cultures were pulsed with 1 μ Ci/well [3H]-thymidine (40-60 Ci/mmole, Amersham, Arlington Heights, IL) and processed with an automated sample harvester onto glass-fiber discs. The discs were allowed to dry and [3H]-thymidine incorporation was determined by counting on a Beckman Scintillation Spectrometer (Model LS6800). Data were calculated as net counts per min (cpm) (mean cpm in stimulated cultures - mean cpm in unstimulated cultures) cpm of unstimulated cultures were consistently $\geq 500$.

Cytotoxic cells: Human allospecific cytotoxic cells were obtained by using the one-way mixed lymphocyte culture reaction established in 24-well plates (Costar No. 3524). PBMC from one donor were used as responder (R) cells and mitomycin C treated (30 min at 37°C followed by three washes in medium) allogeneic PBMC, from an unrelated donor, were used as stimulator (S) cells. Groups of quadruplicate cultures were established with $3 \times 10^6$ R cells cocultured with an equal number of S cells in a final volume of 2 ml medium/well. Various concentrations of TGF-β were added to groups of cultures established in parallel. After seven days of incubation, cells were harvested, washed in medium, and used as effector cells. The cytotoxic activity of these effector cells was determined against lymphoblast targets of the stimulator cell type. Lymphoblast targets were prepared by incubating PBMC ($10 \times 10^6$/10 ml media) with phytohemagglutinin (1 μg/ml), in a T-25 tissue culture flask. Three to four days later, lymphoblasts were harvested, labeled with 150 μCi of $Na_2CrO_4$ (Amersham, Arlington Heights, IL) for 1 hr at 37°C, washed, and used as targets in the cytotoxic cell function assay.

Cytotoxic cell function assay: Triplicates of 0.1 ml volumes containing varying numbers of effector cells were transferred to 96-well, round bottomed microtiter plates (Costar, Cambridge, MA) and followed by the addition of 0.1 ml of 51Cr-labeled lymphoblast targets at $10^4$ cells/well. After 4 h of incubation, the supernatants were harvested (Skatron Inc., Sterling, VA) and the radioactivity was determined using an automatic gamma-counter (model 28037, Micromedic Systems, Inc., Horsham, PA). Specific target lysis expressed as percent cytotoxicity was calculated as follows:

$$\text{Percent cytotoxicity} = \frac{A - B}{C - B} \times 100$$

where A represents the mean count per minute (cpm) in test supernatants (targets cocultured with effectors), B represents the mean cpm of the spontaneous release (targets cultured alone), and C represents the mean cpm of the maximum release (cultures of targets lysed with 1 percent NP-40).

Statistical analysis: The data were analyzed by paired design Student's t test and by analysis of variance for multiple group comparison.

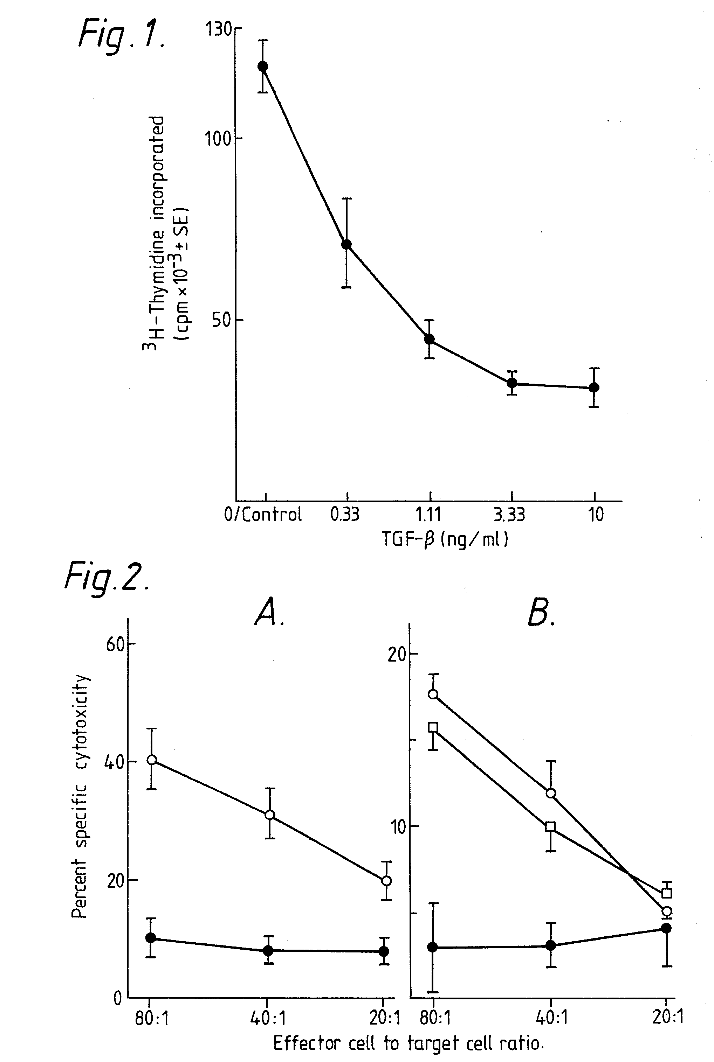

Cells from at least 6 different donors were used to study the effects of TGF-β on PBMC proliferative activities induced by TT. The results of a representative experiment are illustrated in Fig. 1 demonstrating the ability of TGF-β to cause significant inhibition of PBMC proliferative activities in a dose related manner as measured by 3H-thymidine incorporation. The extent of inhibition and the dose response correlation varied from one experiment to the other, however, significant inhibition was consistently observed by the addition of 0.33 ng/ml of TGF-β to the cultures (p<0.01). Further, the extent of inhibition by 10 ng/ml was significantly greater than that caused by 0.33 ng/ml TGF-β in all cases tested.

The influence of various doses of TGF-β on the generation of allospecific cytotoxic cell generation in the mixed lymphocyte reaction (MLR) was examined. In these experiments, TGF-β was added at the time MLR cultures were established. The results of four such experiments (Fig. 2, panel A) show that cells harvested from TGF-β treated MLR cultures (5 ng/ml), and tested against lymphoblast targets of the stimulator cell type, exhibited significantly less cytotoxic activities (p<0.05). The results also show that effector cells from control cultures failed to exhibit cytotoxic activities against lymphoblast targets from a third party indicating the allospecificity of cell activation in the MLR (Fig. 2, panel A).

An independent experiment was performed to determine whether TGF-β effects are manifested during allogeneic cell interaction in the MLR or whether TGF-β interferes with specific cytotoxic cell function against $^{51}$Cr-labeled targets. The results of this experiment show that while effector cells from TGF-β treated MLR cultures exhibited significantly suppressed allospecific cytotoxic activities, no suppression was observed when TGF-β was added at the beginning of the $^{51}$Cr-release assay (Fig. 2, panel B). These results indicate that TGF-β exerts its effect during allogeneic cell interaction in the MLR and does not interfere with the cytolytic activity of sensitized allospecific cytotoxic cells which have been generated in the absence of exogenous TGF-β.

EXAMPLE 2

Effect of TGF-β On Collagenase Secretion by Synovial Fibroblasts

Adherent rheumatoid synovial cells (RSF) were obtained from human synorial tissue and maintained by the method of Amento et al., "J. Clin. Invest." 76:837 (1985).

The effect of TGF-β on IL-1β stimulated collagenase secretion by human RSF was determined by incubating substantially homogeneous acid activated porcine platelet derived TGF-β (R & D Systems) at a concentration of 1 ng/ml to 100 ng/ml with or without 1 ng/ml of Il-1β in RSF culture for 3 days at 38.5°C, after which the collagenase activity in the culture medium was determined by the hydrolysis of 14C collagen. TGF-β had no significant effect in stimulating collagenase from RSF (795 cpm of solubilized 14C- collagen as opposed to the control level of 1,042 cpm), whereas IL-1β strongly activated RSF (4,080 cpm). However, the addition of Il-1β and TGF-β to the RSF culture together resulted in virtually complete inhibition of IL-1β induced activation of RSF collagenase activity (1,591 cpm). The results were quite similar when TNF-α (10 ng/ml) was used in place of Il-1β (the TGF-β concentration in this TNF-α experiment was also 10 ng/ml).

The TNF-α stimulating dose was increased to 100 ng/ml and a dose-response study made against varying concentrations of TGF-β. TGF-β indeed was responsible for the suppression of TNF-α-induced active collagenase generation by synovial fibroblasts, as shown in the following table.

### Table 1

### Dose Response; TNF-α vs. TGF-β

| Additions to RSF | 14C Collagen Solubilization |
|---|---|
| TNF-α (100 ng/ml) + TGF-β (1 ng/ml) | 11,908 ± 1,107 |
| TNF-α (100 ng/ml) + TGF-β (10 ng/ml) | 1,961 ± 33 |
| TNF-α (100 ng/ml) + TGF-β (100 ng/ml) | 2,546 ± 221 |

EXAMPLE 3

Treatment of Patient with Rheumatoid Arthritis

A patient having nondeforming rheumatoid arthritis with multiple joint involvement and a positive latex fixation test for rheumatoid factor had received minimal prior therapy using aspirin and non-steroidal anti-inflammatory agents. The patient presented with an acute flare, notwithstanding prior therapy, and treatment with TGF-β is instituted. Direct intra-articular injections of 50 ng human acid activated TGF-β in istonic saline are made in the left knee, and 500 ng in 10 ml of isotonic saline per day in the right knee, at three day intervals for two weeks, for a total of 5 injections. Less accessible joints are treated by an initial systemic (i.v.) loading infusion of 10 micrograms TGF-β in 5% dextrose, followed by monitoring of serum TGF-β concentrations in order to confirm loading to about 1 ng TGF-β/ml. of serum. Maintenance doses are administered by i.v., i.m. or subcutaneous injections. The patient's response, as measured by diminution of redness and swelling, increased range of articular motion, increased hand grip strength, decreased periods of morning stiffness and favorable serological work up (particularly erythrocyte sedimentation rate), is monitored throughout and dosage adjusted as dictated by clinical progress during the course of therapy as measured by these parameters. The patient's clinical condition is judged to have improved as a result of TGF-β administration.

**0 269 408**

**Claims**

1. The use of transforming Growth Factor-β in the preparation of a medicament for treating an inflammatory disorder.

2. The use according to claim 1 wherein the disorder is accompanied by a Class II or Class III immune response.

3. The use according to claim 2 wherein the disorder is rheumatoid arthritis, systemic lupus erythematosus, or inflammatory bowel disease.

4. The use according to claim 1 wherein medicament is in unit dosage form adapted to provide a therapeutic dosage effective to establish a TGF-β concentration in the inflamed tissue which is greater than about 0.1 ng/cc.

5. The use according to claim 4 wherein the disorder is rheumatoid arthritis and the dosage is effective to establish a TGF-β concentration in the synovial fluid of about from 0.5 to 15 ng/cc.

6. The use according to claim 1 wherein the TGF-β is formulated into a suppository and the disorder is inflammatory bowel disease.

7. The use according to claim 1 wherein the TGF-β is arranged for administration with an antagonist to γ-interferon, an antagonist to TNF-α, an antagonist to TNF-β, an antagonist to IL-1, a gold salt, penicillamine, a nonsteroidal anti-inflammatory agent and/or salicylate.

8. The use according to claim 5 wherein the TGF-β is formulated in a sustained release carrier and administered into a diarthrodial joint.

9. The use according to claim 8 wherein the carrier is a polylactide.

10. The use according to any one of the preceding claims wherein the TGF-β is human TGF-β.

11. A suppository which contains TGF-β.

12. A pharmaceutical preparation comprising TGF-β arranged for simultaneous or sequential administration with a substance selected from an antagonist to γ-interferon, an antagonist to TNF-α, an antagonist to TNF-β, an antagonist to IL-1, a nonsteroidal anti-inflammatory agent, penicillamine and a gold salt.

7

0269408

Fig.1.

Fig.2.

A.

B.

Effector cell to target cell ratio.

Fig.3.

```
h.β-TGF₁:   ALDTNYCFSSTEKNCCVRQLYIDFRKDLGWK-WIHEPKGY
h.β-TGF₃:   ALDTNYCFRNLEENCCVRPLYIDFRQDLGWK-WVHEPKGY
p.β-TGF₃:   ALDTNYCFRNLEENCCVRPLYIDFRQDLGWK-WVHEPKGY
b.β-TGF₂:   ALDAAYCFRRVQDNCCLRPLYIDFKRDLGW----------
            *         *                 *         *
            1         10                20        30


h.β-TGF₁:   HANFCLGPCPYIWSLDT----QYSKVLAL-YNQ--HNPGA
h.β-TGF₃:   YANFCSGPCPYLRSADT----THSTVLGL-YNT--LNPEA
p.β-TGF₃:   YANFCSGPCPYLRSADT----THSSVLGL-YNT--LNPEA
b.β-TGF₂:   
            *         *              *          *
            40        50             60         70


h.β-TGF₁:   SAAPCCVPQALEPLPIVYYV-GRKPKVEQLSNMIVRSCKCS
h.β-TGF₃:   SASPCCMPQDLEPLTILYYV-GRTPKVEQLSNMVVKSCKCS
p.β-TGF₃:   SASPCCVPQDLEPLTILYYV-GRTAKVEQLSNMVVKSCKCS
b.β-TGF₂:   
                      *         *         *         *
                      80        90        100       110
```